# FASCICULE DE BREVET EUROPEEN

(11) **EP 1 077 606 B1**
(45) Date de publication et mention de la délivrance du brevet: **17.12.2003**
(21) Numéro de dépôt: 00909408.7
(22) Date de dépôt: 02.03.2000
(51) Int. Cl.: A01N 37/16, A01N 59/00

(54) **PROCEDE DE PREPARATION D'UNE COMPOSITION ANTIMICROBIENNE**
VERFAHREN ZUR HERSTELLUNG EINER ANTIMIKROBIELLEN ZUSAMMENSETZUNG
METHOD FOR PREPARING AN ANTIMICROBIAL COMPOSITION

(30) Priorité: 05.03.1999 FR 9902927
(43) Date de publication de la demande: 28.02.2001
(73) Titulaire: Laboratoires Anios, 59260 Lille-Hellemmes (FR)
(72) Inventeur: LETARTRE, Bertrand, F-59260 Lille-Hellemmes (FR)
(74) Mandataire: Duthoit, Michel
(86) Numéro de dépôt international: FR0000523
(87) Numéro de publication internationale: WO00053011

(56) Documents cités:
- EP-A- 0 676 499
- FR-A- 2 377 203
- US-A- 3 684 477

## Description

L'invention a pour objet un procédé de préparation d'une composition antimicrobienne ainsi qu'un ensemble de solutions pour la mise en oeuvre d'un tel procédé et une utilisation de la composition antimicrobienne obtenue par ce dernier pour la désinfection d'objets et/ou de surfaces, par exemple des instruments chirurgicaux et/ou endoscopes ou de circuits.

Toutefois, bien que plus particulièrement développée pour de telles applications, l'invention pourra également être utilisée dans tous les domaines, notamment professionnels ou ménagers, dans lesquels on est amené à désinfecter des objets, des surfaces ou autres.

Actuellement, lorsque l'on souhaite utiliser des objets susceptibles de présenter une contamination microbienne, il est connu de les traiter à l'aide de compositions désinfectantes.

A ce jour, on utilise des solutions contenant, entre autres, des aldéhydes, et notamment aldéhyde formique, glyoxalique, glutarique, succinique ou encore orthophtallique.

Ces molécules sont connues pour leur efficacité antimicrobienne. Une pratique d'usage, pour le traitement de l'instrumentation, est de disposer ces solutions dans des bacs partiellement fermés dans lesquels on trempera les objets à traiter.

Toutefois, les produits ci-dessus identifiés ont pour caractéristique physico-chimique d'être extrêmement volatils, d'où un dégagement de vapeurs d'aldéhyde qui entraîne des inconvénients plus ou moins majeurs, selon les procédures et les utilisateurs.

D'autres propositions-sont présentées sous forme de poudre à solubiliser avant l'emploi, ce qui entraîne le risque, notamment, d'aspirer une certaine quantité, non solubilisée, de cette poudre dans des circuits et de les obstruer, au moins partiellement.

D'autres molécules ou associations de molécules ont été proposées, telles que le dioxyde de chlore, le peroxyde d'hydrogène ou des mélanges de péroxyde d'hydrogène, d'acide péracétique et d'acide acétique. Néanmoins ceux-ci ne permettent pas de corriger les phénomènes d'émanations de vapeurs, à partir des bains de trempage, ou n'apportent pas une efficacité suffisante dans un temps de contact acceptable.

Le document EP-A-0676499 concerne un procédé de décoloration de matières textiles à l'aide d'une composition contenant du péroxyde d'hydrogène et un N-acyllactame comme activateur, nécessitant des temps de trempage supérieurs à 30 minutes.

Le document US-A-3684477 concerne une méthode de contrôle de la croissance de microorganismes, en milieu aqueux alcalin, à l'aide de perborate de sodium tétrahydraté, générateur de péroxyde d'hydrogène, et d'un composé organique apte à libérer des radicaux acétyl.

En conséquence, de telles compositions ne sont pas entièrement satisfaisantes lorsqu'elles sont utilisées intensément dans des environnements médicaux, hospitaliers, industriels, ménagers ou autres.

Le but de l'invention est de proposer un procédé de préparation d'une composition antimicrobienne ainsi qu'un ensemble de solutions pour la mise en oeuvre dudit procédé qui pallient les inconvénients précités, tout d'abord en éliminant les difficultés de solubilisation des formes poudres de manière à éviter les risques de dépôt sur les surfaces et dans les circuits.

Un autre but de l'invention est de proposer un procédé de préparation d'une composition antimicrobienne ainsi qu'un ensemble de solutions pour la mise en oeuvre dudit procédé qui permettent de réduire les émanations de principes actifs.

Un autre but de l'invention est de proposer un procédé de préparation d'une composition antimicrobienne ainsi qu'un ensemble de solutions pour la mise en oeuvre dudit procédé qui réduisent les émanations de vapeurs, notamment irritantes, des éventuels autres ingrédients, de départ et/ou d'arrivée, tels que l'acide acétique.

Un autre but de l'invention est de proposer un procédé de préparation d'une composition antimicrobienne ainsi qu'un ensemble de solutions pour la mise en oeuvre dudit procédé qui soient facilement commercialisables grâce à la stabilité desdites solutions pendant une longue durée, dans des conditions de stockage standardisées.

Un autre but de l'invention est de proposer une composition antimicrobienne qui permet, lors de son usage en tant que désinfectant, de diminuer le temps de contact nécessaire à l'obtention d'un traitement efficace, notamment vis-à-vis des formes sporulées de bactéries.

Un autre but de l'invention est de proposer une composition qui soit efficace, dans des limites prédéfinies, lors de son usage en tant que désinfectant, grâce à une stabilité suffisante de son ou ses composants.

Un autre but de l'invention est de proposer une composition qui réduise au mieux, lors de son usage en tant que désinfectant, la réactivité de son ou ses composants avec les métaux ferreux et non ferreux.

Un autre but de l'invention est de proposer une composition dont le pH, lors de son usage en tant que désinfectant, soit le plus proche possible de la neutralité, en l'occurrence faiblement acide ou alcalin.

L'invention concerne tout d'abord un procédé de préparation d'une composition antimicrobienne dans lequel on mélange :
- une base liquide génératrice d'oxygène,
- un générateur liquide de radicaux acétyl, constitué, en poids, de:
   - N-acétylcaprolactame : 1 % à 80 %,
   - alcool: 5 % à 80 %,
   - agents tensio actifs : 0,001 % à 20 %,
   - colorant: 0,001 % à 0,01 %,
   - agents anti-corrosion : 0,001 % à 40 %,
   - dérivé glycolique : 0,001 % à 10 %,
la somme des constituants du générateur liquide étant égale à 100 % en poids.

L'invention concerne également un ensemble de solutions pour la mise en oeuvre d'un tel procédé de préparation d'une composition antimicrobienne comprenant au moins :
- une base liquide génératrice d'oxygène, prévue selon une première dose,
- un générateur liquide de radicaux acétyl, prévu selon une seconde dose, constitué, en poids, de :

- N-acétylcaprolactame : 1 % à 80 %,
- alcool : 5 % à 80 %,
- agents tensio actifs : 0,001 % à 20 %,
- colorant : 0,001 % à 0,01 %,
- agents anti-corrosion : 0,001 % à 40 %,
- dérivé glycolique : 0,001 % à 10 %,
la somme des constituants du générateur liquide étant égale à 100 % en poids.

L'invention concerne en outre une utilisation de la composition antimicrobienne obtenue par le procédé décrit plus haut pour la désinfection. d'objets et/ou de surfaces, par exemple des instruments chirurgicaux et/ou endoscopes ou de circuits.

L'invention sera mieux comprise à la lecture de la description suivante qui en développe certains modes de réalisation.

L'invention concerne tout d'abord un procédé de préparation d'une composition antimircrobienne dans lequel on mélange :
- une base liquide génératrice d'oxygène,
- un générateur liquide de radicaux acétyl, notamment labils, constitué au moins d'acétylcaprolactam.

L'utilisation de forme liquide permet ainsi d'éviter les inconvénients des poudres et les composés choisis permettent d'éviter les émanations de produits actifs et/ou de vapeurs irritantes.

Ledit procédé de préparation d'une composition antimicrobienne pourra être mis en oeuvre, de manière avantageuse, extemporanément.

Ledit mélange de la base et du générateur est prévu, notamment, apte à permettre l'obtention, essentiellement, d'acide péracétique, par réaction de perhydrolyse.

Selon l'invention, on pourra éventuellement prévoir dans ladite base et/ou dans ledit générateur et/ou on pourra éventuellement ajouter dans ladite composition, après ou avant le mélange de ladite base et dudit générateur, des agents tensio-actifs, aptes à renforcer l'activité antimicrobienne et/ou la solubilité du générateur, des agents anti-corrosions, des agents régulateurs de pH et/ou des tiers solvants, de type alcool et/ou glycol.

Plus précisément, la base est constituée, par exemple, d'un agent oxydant liquide, par exemple, de type péroxyde d'hydrogène.

Ladite base est ainsi formulée, notamment, à partir au moins de péroxyde d'hydrogène stabilisé, par exemple à un pH donné.

Ladite base sera plus ou moins concentrée selon qu'elle sera prête à l'emploi après addition du générateur ou à diluer avec une eau de qualité adéquate, avant usage et après ou avant addition du générateur.

On prévoit, par exemple, une quantité de péroxyde d'hydrogène, apte à renforcer l'activité antimicrobienne et à provoquer la synthèse de l'acide péracétique après mélange de ladite base et dudit générateur. Il s'agit, notamment, d'une quantité d'au moins 0,25 % de péroxyde d'hydrogène.

Ladite base contient en outre, éventuellement, des agents stabilisants et/ou régulateurs de pH, afin de stabiliser le peroxyde d'hydrogène lors du stockage, de stabiliser le mélange dans le temps et/ou d'intervenir dans la cinétique de la réaction de perhydrolyse lors du mélange de la base et du générateur. L'ajustement du pH de la base sera ainsi réalisé, notamment, afin de produire la quantité désirée d'acide péracétique ou d'autres composés antimicrobiens, dans un temps donné, le plus court possible.

Lesdits agents stabilisants et/ou régulateurs de pH sont constitués, par exemple, des composés alcalins de type hydroxyde de sodium, hydroxyde de potassium et/ou autres. Il pourra encore s'agir, notamment, d'acides de type minéraux ou organiques, avec effet tampon si nécessaire, et/ou de composés de type monoéthanolamine, diethanolamine, triethanolamine et/ou autres.

La base contient en outre, éventuellement, des agents anti-corrosions. Ceux-ci sont choisis pour leur capacité à réduire les différentes formes de corrosion des métaux ferreux et non ferreux.

Il s'agit, par exemple, de phosphates, de phosphonates, de dérivés triazoliques et/ou autres.

La base pourra encore contenir, éventuellement, un réactif ou indicateur coloré et/ou un colorant qui réagit par changement de couleur, notamment lors de l'addition du générateur.

A ce sujet, le générateur est constitué, comme déjà dit, au moins d'acétylcaprolactam. Il s'agit, par exemple, de N acétylcaprolactam. Il permet la libération de radicaux acétyl oxydables lors du mélange.

Dans le cas de production d'acide péracétique, la quantité de générateur dudit acide ajouté à la base détermine le taux d'acide péracétique produit lors du mélange de la base et du générateur.

Au générateur, seront éventuellement incorporés différentes familles de composés dans le but d'optimiser un certain nombre de données.

A titre d'exemple, il pourra être prévu d'incorporer des agents tensio-actifs, de manière à renforcer l'activité antimicrobienne et une meilleure pénétration du produit au sein des corps creux et des anfractuosités. De tels produits pourront également améliorer la dissolution du composé générateur de radicaux acétyl employé tel que notamment le N acétylcaprolactam.

Lesdits agents tensio-actifs seront, par exemple, de type non ionique, comme notamment des condensats d'oxyde d'éthylène ou propylène, des alkytpolyglucosides ou des alcools gras éthoxylés et/ou propoxylés. Lesdits agents tensio-actifs pourront encore être, par exemple, de type anionique comme notamment les sulfates, les sulfonates ou sulfosuccinates, ou de type amphotérique ou cationique.

Il pourra en outre être éventuellement prévu dans le générateur des composés favorisant la dispersion et/ou la solubilité du générateur dans la base. Il s'agit, par exemple, d'alcools aliphatiques ou cycliques, tels que l'éthanol, l'isopropanol, le n-propanol, le butanoi, mais aussi des dérivés glycoliques, tels que le monoéthylèneglycol, monopropylèneglycol, butylglycol et/ou autres.

Le générateur pourra en outre contenir, éventuellement, des agents colorants et/ou des indicateurs colorés, qui réagissent lors de l'addition du générateur dans la base.

A titre d'exemple, les proportions, en poids, des différents constituants de la base et du générateur conformes à l'invention pourront être les suivants :
Base :
   - Peroxyde d'hydrogène : 0,5 % - 35 %, notamment 2 à 4 %
   - Stabilisants : 0,0001 % - 5 %, notamment 0,0005 à 0.009 %
   - Agents anti-corrosion: 0,001 % - 2 %, notamment 0,04 à 0,06 %
   - Régulateur de pH : 0,001 à 5 %, notamment 0,01 à 1 %
   - Eau : qsq 100 %.
Générateur :
   - n Acetylcaprolactam : 1 % - 80 %, notamment 40 à 60 %
   - Alcool : 5 % - 80 %, notamment 30 à 40 %
   - Agents tensio actifs : 0,001 % - 20 %, notamment 4 à 7 %
   - Colorant : q.s., notammetn 0,001 à 0,01 %
   - Agents anti-corrosion: 0,001 % - 40 %, notamment 0,001 à 0,01 %
   - Dérivé glycolique : 0,001 % - 10 %, notamment 0,5 à 1 %.

A titre d'exemple, le pH de la base est fixé entre 6 et 8 ± 0,1.

Un tel dosage des ingrédients favorise une production maîtrisée d'acide péracétique, une stabilité de l'acide péracétique produit, et donc une efficacité antimicrobienne disposant d'un spectre d'activité au moins sur les bactéries, les levures et moisissures, les virus et les spores de bactéries, ceci dans un temps de contact court.

L'invention concerne également un ensemble de solutions pour la mise en oeuvre du procédé décrit plus haut. Cet ensemble comprend au moins :
- une base liquide génératrice d'oxygène, prévue selon une première dose,
- un générateur liquide de radicaux acétyl, prévu selon une seconde dose.

Lesdites première et seconde doses sont réparties, par exemple, dans un premier et un second récipients.

Pour mettre en oeuvre le procédé de préparation de la composition antimicrobienne, il pourra alors suffir de mélanger le contenu desdits récipients, prévu prêt à l'emploi et/ou prédosé.

L'invention concerne en outre l'utilisation de la composition antimicrobienne obtenue par le procédé décrit plus haut pour la désinfection d'objets et/ou de surfaces, par exemple, des instruments chirurgicaux et/ou endoscopes ou de circuits.

Naturellement, d'autres mises en oeuvre et/ou applications de l'invention, à la portée de l'Homme de l'art, auraient pu être envisagées sans pour autant sortir du cadre de l'invention.

## Revendications

1. Procédé de préparation d'une composition antimicrobienne dans lequel on mélange :
- une base liquide génératrice d'oxygène,
- un générateur liquide de radicaux acétyl, constitué, en poids, de :
- N-acétytcaprotactame : 1 % à 80 %,
- alcool : 5 % à 80 %,
- agents tensio actifs : 0,001 % à 20 %,
- colorant : 0,001 % à 0,01 %,
- agents anti-corrosion : 0,001 % à 40 %,
- dérivé glycolique : 0,001 % à 10 %,
la somme des constituants du générateur liquide étant égale à 100 % en poids.

2. Procédé de préparation d'une composition antimicrobienne selon la revendication 1, mis en oeuvre extemporanément.

3. Procédé selon la revendication 1, dans lequel le mélange de la base et du générateur est prévu apte à permettre l'obtention, essentiellement, d'acide péracétique, par réaction de pérhydrolyse.

4. Procédé selon la revendication 1, dans lequel la base est formulée à partir au moins de péroxyde d'hydrogène stabilisé.

5. Procédé selon la revendication 1, dans lequel on prévoit dans la base au moins 0,25 % de péroxyde d'hydrogène.

6. Procédé selon la revendication 1, dans lequel la base liquide génératrice d'oxygène est constituée, en poids, de :
- péroxyde d'hydrogène : 0,5 % à 35 %,
- stabilisants : 0,0001 % à 5 %,
- agents anti-corrosion : 0,001 % à 2 %,
- régulateurs de pH : 0,001 % à 5 %,
- eau : qsp 100 %.

7. Ensemble de solutions pour la mise en oeuvre du procédé de préparation d'une composition antimicrobienne selon la revendication 1, comprenant au moins :
- une base liquide génératrice d'oxygène, prévue selon une première dose,
- un générateur liquide de radicaux acétyl, prévu selon une seconde dose, constitué, en poids, de :
- N-acétylcaprolactame : 1 % à 80 %,
- alcool : 5 % à 80 %,
- agents tensio actifs : 0,001 % à 20 %,
- colorant : 0,001 % à 0,01 %,
- agents anti-corrosion : 0,001 % à 40 %,
- dérivé glycolique : 0,001 % à 10 %,
la somme des constituants du générateur liquide étant égale à 100 % en poids.

8. Utilisation de la composition antimicrobienne obtenue par le procédé selon la revendication 1, pour la désinfection d'objets et/ou de surfaces, par exemple des instruments chirurgicaux et/ou endoscopes ou de circuits.

## Patentansprüche

1. Verfahren zur Zubereitung einer antimikrobiellen Zusammensetzung in der folgendes gemischt wird :
eine sauerstofferzeugende, flüssige Base,
ein flüssiger Generator von Azetylradikalen, der gewichtsmäßig besteht aus :
N-Azetylcaprolaktam : 1 % bis 80 %,
Alkohol : 5 % bis 80 %,
Oberflächenaktiven Mitteln : 0,001 % bis 20 %,
Farbstoff : 0,001 % bis 0,01 %,
Korrosionsschutzmitteln : 0,001 % bis 40 %,
Glykolderivat : 0,001 % bis 10 %,
wobei die Summe der Bestandteile des flüssigen Generators gleich 100 Gew.% ist.

2. Verfahren zur Zubereitung einer antimikrobiellen Zusammensetzung nach Anspruch 1, das erst bei Bedarf angewandt wird.

3. Verfahren nach Anspruch 1, bei dem die Mischung der Base und des Generators geeignet vorgesehen ist, das Erhalten von hautpsächlich Peressigsäure durch Perhydrolyse zu ermöglichen.

4. Verfahren nach Anspruch 1, bei dem die Base ab wenigstens stabilisiertem Wasserstoffperoxid formuliert wird.

5. Verfahren nach Anspruch 1, bei dem in der Base wenigstens 0,25 % Wasserstoffperoxid vorgesehen wird.

6. Verfahren nach Anspruch 1, bei dem die flüssige sauerstofferzeugende Base gewichtsmäßig besteht aus :
Wasserstoffperoxid : 0,5 % bis 35 %,
Stabilisatoren : 0,0001 % bis 5 %,
Korrosionsschutzmitteln : 0,001 % bis 2 %,
pH-Regulatoren : 0,001 % bis 5 %,
Wasser: Rest bis 100 %.

7. Gesamtheit von Lösungen zur Anwendung des Verfahrens zur Zubereitung einer antimikrobiellen Zusammensetzung nach Anspruch 1, umfassend wenigstens :
eine nach einer ersten Dosis vorgesehene, flüssige sauerstofferzeugende Base,
einen nach einer zweiten Dosis vorgesehenen, flüssigen Generator von Azetylradikalen, der gewichtsmäßig besteht aus :
N-Azetylcaprolaktam : 1 % bis 80 %,
Alkohol: 5 % bis 80 %,
Oberflächenaktiven Mitteln : 0,001 % bis 20 %,
Farbstoff: 0,001 % bis 0,01 %,
Korrosionsschutzmitteln : 0,001 % bis 40 %,
Glykolderivat : 0,001 % bis 10 %,
wobei die Summe der Bestandteile des flüssigen Generators gleich 100 Gew.% ist.

8. Verwendung der durch das Verfahren nach Anspruch 1 erhaltenen antimikrobiellen Zusammensetzung für die Entseuchung von Gegenständen und/oder Oberflächen, z.B. von chirurgischen und/oder endoskopischen Instrumenten oder von Schaltungen.

## Claims

1. A method for preparing an antimicrobial composition, wherein the following elements are mixed:
- an oxygen generating liquid base,
- an acetyl radical liquid generator, consisting of, in weight:
- N-acetlycaprolactam: 1 to 80%,
- alcohol: 5 to 80%,
- surfactants: 0.001 to 20%,
- colouring agent: 0.001 to 0.01 %,
- anticorrosion agents: 0.001 to 40%,
- glycol derivate: 0.001 to 10 %,
the sum of the constituents of the liquid generator being equal to 100% in weight.

2. A method for preparing an antimicrobial composition according to claim 1**,** implemented extemporaneously.

3. A method according to claim 1, wherein the mixture of the base and of the generator is provided as .capable of obtaining, essentially, peracetic acid, by perhydrolysis reaction.

4. A method according to claim 1, wherein the base is formulated from at least stabilised hydrogen peroxide.

5. A method according to claim 1, wherein at least 0.25 % hydrogen peroxide is provided in the base.

6. A method according to claim 1, wherein the oxygen generating liquid base consists of, in weight:
- hydrogen peroxide: 0.5 to 35%,
- stabilisers: 0.0001 to 5 %,
- anticorrosion agents: 0.001 to 2%,
- pH regulators: 0.001 to 5%,
- water: qsp 100%.

7. A set of solutions for implementing the method for preparing an antimicrobial composition according to claim 1, comprising at least:
- an oxygen generating liquid base, provided as a first dose,
- an acetyl radical liquid generator, provided as a second dose, consisting of, in weight:
- N-acetlycaprolactam: 1 to 80%,
- alcohol: 5 to 80%,
- surfactants: 0.001 to 20%,
- colouring agent: 0.001 to 0.01 %,
- anticorrosion agents: 0.001 to 40%,
- glycol derivate: 0.001 to 10 %,
the sum of the constituents of the liquid generator being equal to 100% in weight.

8. Usage of the antimicrobial composition obtained by the method according to claim 1, for disinfection of objects and/or surfaces, for example surgical instruments and/or endoscopes or circuit.
